# EUROPÄISCHE PATENTSCHRIFT

(11) **EP 2 881 723 B1**
(45) Veröffentlichungstag und Bekanntmachung des Hinweises auf die Patenterteilung: **07.06.2017**
(21) Anmeldenummer: 14196245.6
(22) Anmeldetag: 04.12.2014
(51) Int. Cl.: G01M 99/00, G01N 33/36

(54) **Prüfungsapparatur für Schutzkleidung**
Testing equipment for safety clothing
Appareil de vérification pour vêtement de protection

(30) Priorität: 06.12.2013 DE 102013225190
(43) Veröffentlichungstag der Anmeldung: 10.06.2015
(73) Patentinhaber: Bundesrep. Deutschland, v.d. d. Bundesministerium des Innern,v.d.das Bundesamt f. Bevölkerungsschutz und Katastrophenhilfe, v.d. den Präsidenten, 53127 Bonn (DE)
(72) Erfinder: Arnold, Wolfram, 53177 Bonn (DE); Bachmann, Udo, 53225 Bonn (DE); Trebbe, Roman, 53913 Swisttal-Odendorf (DE); Winkler, Iris, 53424 Remagen (DE); Drobig, Matthias, 53913 Swisttal-Buschhoven (DE)
(74) Vertreter: dompatent von Kreisler Selting Werner - Partnerschaft von Patent- und Rechtsanwälten mbB

(56) Entgegenhaltungen:
- EP-A1- 1 580 544
- US-A- 4 468 951
- US-A- 5 887 477

## Beschreibung

Die Erfindung betrifft eine Prüfungsapparatur zur Überprüfung der Rückhaltefähigkeit von Schutzkleidungsmaterialien gegenüber chemischen Stoffen, insbesondere zur Überprüfung neuartiger lichtaktiver Schutzmaterialien.

In bisherigen Prüfungen von Schutzkleidungsmaterialien auf deren Rückhaltefähigkeit gegenüber chemischen Stoffen werden Glasapparaturen verwendet, in denen eine Probe des Schutzkleidungsmaterials in einen Prüfling eingespannt wird. Auf die beim Tragen außen liegende Seite des Prüflings (Außenseite) wird eine definierte Konzentration ausgewählter chemischer Substanzen auf das Material geführt. Hierzu weisen die üblichen Prüfungsapparaturen mindestens zwei Anschlüsse auf, über die die chemischen Substanzen zugeführt werden und wieder abgeführt werden. Auf der beim Tragen innen liegenden Seite des Prüflings (Innenseite) werden Luftproben genommen, um Durchbruchszeit sowie Art und Menge der durchgebrochenen Chemikalien zu detektieren.

Durch die Anordnung der Zu- und Ableitungen der bestehenden Apparatur kann das Überströmen des Prüflings nur bedingt erfolgen, da der Gasstrom mehr oder weniger direkt, aber nicht zentriert, auf den Prüfling gerichtet wird. Dies ist für Untersuchungen mit chemischen Kampfstoffen durchaus gewünscht. Bei Untersuchungen, die Prüfbedingungen simulieren sollen wie sie in zivilen Einsatzszenarien vorkommen, können mit dieser Anordnung zu starke Beaufschlagungen des Prüflings erzeugt werden. Auch durch die Ausführung der Prüfapparatur in Glas besteht eine erhöhte Bruch- und Verletzungsgefahr. Hierdurch werden das Auseinandernehmen der Prüfungsapparatur, das Einsetzen eines neuen Prüflings und das Wiederzusammenbauen für einen erneuten Test erheblich erschwert. Auch ist üblicherweise eine vertikale Fixierung des Prüflings in der Prüfungsapparatur vorgesehen, was den Auf- und Abbau der Apparatur weiter erschwert, so dass Reihenversuchte zeitlich aufwändig werden.

US 4,468,951 beschreibt eine Prüfungsapparatur, bei der der Prüfling zwischen zwei Gehäuseteilen fixiert wird, so dass sowohl über dem Prüfling als auch unter dem Prüfling eine Kammer gebildet wird, die mit Anschlüssen verbunden ist. Dabei ist das erste Gehäuseteil identisch mit dem zweiten Gehäuseteil ausgebildet und diese werden durch Endplatten miteinander fixiert.

US 5,887,477 beschreibt eine Demonstrationsvorrichtung zur Demonstration der Wasserdichtigkeit und der Atmungsaktivität eines Stoffes. Die Demonstrationsvorrichtung besteht aus einem Kunststoff und weist transparente Seitenwände auf, um einem Publikum sowohl die Wasserdichtigkeit als auch die Atmungsaktivität zu demonstrieren.

Darüber hinaus sind neuartige Schutzkleidungsmaterialien in der Entwicklung, die einen Katalysator enthalten, der bei Bestrahlung mit Licht bestimmter Wellenlänge chemische Stoffe zu idealerweise ungiftigen Stoffen umwandelt und so zusätzlich zur Absorptionswirkung zum Schutz der Einsatzkraft im Schutzanzug beiträgt. Die Überprüfung der Rückhaltefähigkeit dieser neuartigen absorbierenden Schutzkleidungmaterialien müssen daher unter Einwirkung von Licht durchgeführt werden können. Allerdings weisen die bekannten Prüfungsapparaturen den Nachteil auf, dass die verwendeten Glasapparaturen nicht durchlässig sind für alle Wellenlängen des Lichts und insbesondere im UV-Bereich eine erhebliche unerwünschte Absorption stattfinden kann. Darüber hinaus werden durch die Anordnung der Anschlüsse erhebliche Teile des zu prüfenden Schutzkleidungsmaterials verschattet, so dass eine effektive und gleichbleibende Beleuchtung mit den bisher bekannten Prüfungsapparaturen nicht möglich ist.

Aufgabe der Erfindung ist es, eine Prüfungsapparatur zu schaffen, die einfach zu handhaben ist und mit der lichtaktive Schutzkleidungsmaterialien geprüft werden können.

Die Lösung der Aufgabe erfolgt erfindungsgemäß durch eine Vorrichtung gemäß Anspruch 1.

Die erfindungsgemäße Prüfungsapparatur zur Prüfung der Rückhaltefähigkeit von Schutzkleidungsmaterialien weist ein erstes Gehäuseteil auf, durch welches ein erster Hohlraum ausgebildet wird. Dabei ist das erste Gehäuseteil insbesondere zylindrisch ausgebildet, wodurch eine einfache Bauweise gewährleistet wird. Die erfindungsgemäße Prüfungsapparatur weist ein zweites Gehäuseteil auf, durch welches ein zweiter Hohlraum ausgebildet wird. Dabei ist das zweite Gehäuseteil insbesondere im Wesentlichen identisch mit dem ersten Gehäuseteil hinsichtlich der vorgesehenen Form und den verwendeten Materialien. Der erste Hohlraum gebildet durch das erste Gehäuseteil ist von dem zweiten Hohlraum gebildet durch das zweite Gehäuseteil durch einen Prüfling getrennt. Der Prüfling enthält dabei das zur Überprüfung vorgesehene Schutzkleidungsmaterial. Durch den Prüfling wird der erste Hohlraum insbesondere gasdicht vom zweiten Hohlraum getrennt, so dass kein direktes Überströmen der eingeleiteten chemischen Substanz vom ersten Hohlraum zum zweiten Hohlraum ohne Durchdringen des Prüflings erfolgen kann. Der Prüfling ist dabei mit einer Befestigungsvorrichtung mit mindestens einem Befestigungselement zwischen dem ersten Gehäuseteil und dem zweiten Gehäuseteil insbesondere klemmend fixiert.

Erfindungsgemäß ist das erste Gehäuseteil zumindest teilweise lichtdurchlässig, insbesondere UV-durchlässig. Somit ist das erste Gehäuseteil in einem bestimmten Bereich lichtdurchlässig, so dass von einer externen Lichtquelle Licht einer bestimmten Wellenlänge auf das zu prüfende Schutzkleidungsmaterial gerichtet werden kann. Insbesondere ist das erste Gehäuseteil zylindrisch ausgestaltet. Erfindungsgemäß ist die dem Prüfling gegenüberliegende Endfläche zumindest teilweise durch ein transparentes Material ausgebildet, bei dem es sich t um Quarzglas handelt. Dabei hat Quarzglas den Vorteil im ultravioletten Bereich des Spektrums noch ausreichend transparent zu sein. So kann Licht insbesondere durch eine Endfläche des vorzugsweise zylindrisch ausgestalteten ersten Gehäuseteils in den ersten Hohlraum eintreten, diesen der Länge nach durchlaufen und besonders bevorzugt, rechtwinklig auf das zu prüfende Schutzkleidungsmaterial auftreffen. Insbesondere wird hierdurch die gesamte den chemischen Substanzen ausgesetzte Fläche des zu prüfenden Schutzkleidungsmaterials gleichmäßig beleuchtet.

Insbesondere umfasst der ultraviolette Bereich des Spektrums die Wellenlängen von 100 - 380 nm, gemäß DIN 5031-7. Und vorzugsweise ist das erste Gehäuseteil zumindest teilweise transparent für den sichtbaren Bereich und/oder den UV-Bereich von 250 - 365 nm und besonders bevorzugt für den gesamten UV-Bereich wie vorstehend definiert.

Vorzugsweise ist das erste Gehäuseteil und besonders bevorzugt auch das zweite Gehäuseteil aus einem nicht-transparenten, also opaken Material hergestellt, sodass nur ein kleiner Bereich des ersten Gehäuseteils und insbesondere die dem Prüfling gegenüberliegende Endfläche des ersten Gehäuseteils transparent ist für sichtbares Licht und/oder UV-Strahlung. Insbesondere ist weder das erste Gehäuseteil noch das zweite Gehäuseteil vollständig aus Glas gefertigt. Dies hätte in beiden Fällen den Nachteil, dass bei der Bestrahlung des Prüflings mit dem Licht es zu Reflexionen kommt, die ebenfalls die Unterseite des Prüflings beleuchten würden, was für ein reproduzierbares Prüfverfahren nachteilig ist und vermieden wird.

Insbesondere weist die Prüfungsapparatur ausschließlich ein erstes Gehäuseteil, ein zweite Gehäuseteil, einen Prüfling und eine Befestigungsvorrichtung auf. Hierdurch wird der Aufbau der Prüfungsapparatur wesentlich vereinfacht, wodurch eine wesentliche Zeiteinsparung beim Auf- und Abbau der Prüfungsapparatur erreicht werden kann.

Da die Prüfungsapparatur zur Prüfung der Rückhaltefähigkeit von Schutzkleidungsmaterialien gegenüber chemischer Substanzen vorgesehen ist, ist insbesondere das erste Gehäuseteil und/oder das zweite Gehäuseteil aus einem chemisch inerten Material hergestellt. Hierdurch wird sichergestellt, dass die chemischen Substanzen innerhalb der Prüfungsapparatur eingeschlossen werden können. Vorzugsweise handelt es sich bei dem chemisch inerten Material um einen Kunststoff, wie z.B. Polytetrafluorethen. Dadurch ist sichergestellt, dass ein Zerbrechen der Gehäuseteile nicht möglich ist. Gleichzeitig ist die Herstellung von Kunststoffteilen deutlich kostengünstiger als die Herstellung der Prüfungsapparatur aus Glas oder insbesondere Quarzglas.

Insbesondere, falls das erste Gehäuseteil und/oder das zweite Gehäuseteil aus einem chemisch inerten Kunststoff hergestellt werden, ist dieser vorzugsweise nicht transparent bzw. opak.

Das erste Gehäuseteil und/oder das zweite Gehäuseteil weisen insbesondere mindestens zwei Anschlüsse als Zuleitungen und Ableitungen auf. Über eine Zuleitung zum ersten Gehäuseteil wird eine ausgewählte chemische Substanz oder ein Gemisch ausgewählter chemischer Substanzen in den ersten Hohlraum der Prüfungsapparatur eingeleitet. Über die Ableitung werden die chemischen Substanzen aus dem ersten Hohlraum ausgeleitet. Insbesondere können mehr als eine Ableitung vorgesehen sein. Diese zusätzliche Ableitung bewirkt eine Vergrößerung des Ausströmquerschnitts zur Verhinderung eines im Prüfverfahren unerwünschten Staudrucks im ersten Hohlraum. Mit der Zu- und Ableitung des zweiten Gehäuseteils ist vorzugsweise ein Prüfzyklus verbunden, so dass über die Ableitung Luftproben aus dem zweiten Hohlraum des zweiten Gehäuseteils entnommen werden können und über eine geeignete Prüfeinrichtung die Durchbruchszeit sowie Art und ggf. Menge der durchgebrochenen Substanz detektiert werden können.

Insbesondere weist das erste Gehäuseteil zusätzlich oder alternativ zu den oben genannten mindestens zwei Anschlüssen einen auf die Mitte des Prüflings gerichteten Anschluss auf. Durch diesen Anschluss kann ein Gasstrom direkt auf den Prüfling gerichtet werden. Dabei ist der Gasstrom zentriert auf den Prüfling. Vorzugsweise wird hierzu der auf die Mitte des Prüflings gerichtete Anschluss in den ersten Hohlraum fortgesetzt, so dass der Anschluss kurz vor oder in kleinem Abstand über der Mitte des Prüflings endet. Dabei ist jedoch der Abstand zum Prüfling nicht wesentlich, so dass auch ein kürzerer Anschluss die Aufgabe erfüllen kann, einen Gasstrom auf die Mitte des Prüflings zu richten. Hierbei kann es insbesondere vorgesehen sein, dass dieser Anschluss eine Düse aufweist zur weiteren Konzentration des Gasstroms auf die Mitte des zu prüfenden Schutzkleidungsmaterials.

Insbesondere ist das erste Gehäuseteil oberhalb vom Prüfling und ebenso oberhalb vom zweiten Gehäuseteil angeordnet, wobei zusätzlich der Prüfling oberhalb vom zweiten Gehäuseteil angeordnet ist. Hierdurch ergibt sich eine aufrechte Anordnung der Prüfungsapparatur, so dass die wesentlichen Teile der Prüfungsapparatur von oben nach unten in der Reihenfolge erstes Gehäuseteil, Prüfling, zweites Gehäuseteil angeordnet sind. Hierdurch ergibt sich ein vereinfachter Aufbau, da lediglich das erste Gehäuseteil vom Prüfling entfernt werden muss, um den Prüfling auszutauschen. Dabei kann der Prüfling weiterhin auf dem zweiten Gehäuseteil aufliegen. Selbst wenn der Prüfling zum Austauschen des zu überprüfenden Schutzkleidungsmaterials komplett aus der Prüfungsapparatur herausgenommen werden muss, so kann bei der aufrechten Anordnung der Prüfungsapparatur zumindest das zweite Bauteil unverändert bleiben. Somit reduziert sich die Anzahl der erforderlichen Schritte für den Auf- und Abbau der Prüfungsapparatur.

Insbesondere ist das mindestens eine Befestigungselement der Befestigungsvorrichtung flexibel ausgestaltet, so dass der Prüfling unterschiedliche Dicken aufweisen kann und trotzdem eine sichere und insbesondere klemmende Fixierung des Prüflings zwischen dem ersten Gehäuseteil und dem zweiten Gehäuseteil erfolgen kann. Da mit der Prüfungsapparatur insbesondere unterschiedliche Schutzkleidungsmaterialien geprüft werden sollen und diese unter Umständen unterschiedliche Dicken aufweisen, wird durch ein flexibles Befestigungselement verhindert, dass für jede Dicke des Schutzkleidungsmaterials eine gesonderte Befestigung vorgesehen werden muss. Dies vereinfacht den Aufbau und reduziert die Anzahl der für eine Prüfung erforderlichen Teile. Erfindungsgemäß weist die Befestigungsvorrichtung eine das erste Gehäuseteil zumindest teilweise überdeckende zumindest teilweise lichtdurchlässige Abdeckung auf. Dabei ist die Abdeckung insbesondere UV-durchlässig. Dabei ist die Abdeckung lichtdurchlässig in einem Bereich, welcher zumindest teilweise dem lichtdurchlässigen Bereich des ersten Gehäuseteils entspricht. Somit kann das zu prüfende Schutzkleidungsmaterial trotz Vorsehen eine Abdeckung weiter mit Licht beleuchtet werden. Vorzugsweise ist die Abdeckung einteilig mit dem ersten Gehäuseteil verbunden, wodurch sich die Anzahl der Bauteile weiter reduziert.

Insbesondere ist das mindestens eine Befestigungselement der Befestigungsvorrichtung direkt mit dem ersten Gehäuseteil und/oder dem zweiten Gehäuseteil verbunden, bevorzugt jedoch mit der Abdeckung. Vorzugsweise weist die Prüfungsapparatur zusätzlich ein Basiselement auf. Mit diesem Basiselement ist das Befestigungselement insbesondere ebenfalls verbunden. Bei dem Befestigungselement kann es sich beispielsweise um Schrauben, Zugfedern u.dgl. handeln. Dadurch, dass das Befestigungsmittel bevorzugt sowohl mit der Abdeckung als auch mit dem Basiselement verbunden ist, kann der Prüfling zwischen dem ersten Gehäuseteil und dem zweiten Gehäuseteil über das Befestigungselement klemmend fixiert werden. Die Fixierung kann dabei insbesondere schraubzwingenartig erfolgen, so dass die Klemmung des Prüflings durch ein Festziehen des Befestigungselements verstärkt wird, zum Abbau der Prüfungsapparatur jedoch lediglich das Befestigungselement gelöst werden muss. Insbesondere ist dabei der Abstand zwischen Abdeckung und Basiselement variabel, so dass ein Prüfling mit unterschiedlicher Dicke zwischen dem ersten Gehäuseteil und dem zweiten Gehäuseteil klemmend fixiert werden kann.

Vorzugsweise sind das zweite Gehäuseteil und das Basiselement einteilig ausgeführt. Insbesondere können dann die Anschlüsse des zweiten Gehäuseteils durch das Basiselement geführt werden. Hierdurch wird der Aufbau der Prüfungsapparatur weiter vereinfacht.

Besonders bevorzugt ist es dabei, dass die Prüfungsapparatur ausschließlich ein erstes Gehäuseteil, ein zweites Gehäuseteil, einen Prüfling, ein Basiselement und eine Befestigungsvorrichtung aufweist. Somit wird eine einfach zu handhabende und in der Herstellung kostengünstige Prüfungsapparatur geschaffen, die es ermöglicht, Schutzkleidungsmaterialien zu prüfen, die durch Licht angeregte photokatalytische Substanzen nutzen, um Gefahrenstoffe zersetzen zu können.

Als weiteres Bauteil weist die Prüfungsapparatur insbesondere eine Dichtungsmanschette auf, die das erste Gehäuseteil mit dem zweiten Gehäuseteil verbindet und dabei den Prüfling vollständig überdeckt. Die Dichtungsmanschette schließt dabei dicht sowohl mit dem ersten Gehäuseteil als auch mit dem zweiten Gehäuseteil ab. Eventuell am Querschnitt des Prüflings austretende chemische Substanzen werden hierdurch sicher von der Umgebung abgekapselt. Die Dichtungsmanschette stellt dabei ein weiteres Bauteil dar, so dass die Prüfungsapparatur vorzugsweise ausschließlich ein erstes Gehäuseteil, ein zweites Gehäuseteil, einen Prüfling, eine Befestigungsvorrichtung und zusätzlich eine Dichtungsmanschette aufweist. Optional weist diese Prüfungsapparatur darüber hinaus noch ein Basiselement auf. Weitere Bauteile sind nicht erforderlich.

Nachfolgend wird die Erfindung anhand einer bevorzugten Ausführungsform näher erläutert.

Es zeigen:
- Fig. 1: eine Schnittansicht einer Prüfungsapparatur entlang der I-I Linie der Fig. 2 und
- Fig. 2: eine Draufsicht auf die Prüfungsapparatur gezeigt in Fig. 1.

Die erfindungsgemäße Prüfungsapparatur weist ein erstes Gehäuseteil 10 auf, durch welches ein Hohlraum 12 gebildet wird. Das erste Gehäuseteil 10 ist dabei oberhalb von einem Prüfling 14 angeordnet. Der Prüfling 14 weist dabei das zu untersuchende Schutzkleidungsmaterial 16 auf. Der Prüfling 14 ist dabei oberhalb von einem zweiten Gehäuseteil 18 angeordnet. Durch das zweite Gehäuseteil 18 wird ein zweiter Hohlraum 20 ausgebildet. Dabei ist der erste Hohlraum 12 vom zweiten Hohlraum 20 durch den Prüfling 14 voneinander getrennt. Sowohl das erste Gehäuseteil 10 als auch das zweite Gehäuseteil 18 schließen dabei dicht mit dem Prüfling 14 ab. Hierzu sind Dichtungen 22 vorgesehen.

Weiter ist in der Ausführungsform ein Basiselement 24 gezeigt. Das Basiselement 24 schließt dabei das dem Prüfling 14 abgewandte Ende des zweiten Gehäuseteils 18 ab. Wiederum sind zwischen dem zweiten Gehäuseteil 18 und dem Basiselement 24 Dichtungen 26 vorgesehen, um den Hohlraum 20 gas- und flüssigkeitsdicht auszubilden. Alternativ können das Basiselement 24 und das zweites Gehäuseteil 18 als ein zusammenhängendes Bauteil ausgeführt sein. Dann entfällt die Notwendigkeit der Dichtungen 26.

Das dem Prüfling 14 gegenüberliegende Ende des ersten Gehäuseteils 10 ist durch ein Quarzglas 28 abgeschlossen. Dabei ist die Verbindung zwischen dem Quarzglas 28 und dem erstem Gehäuseteil 10 vorzugsweise dauerhaft insbesondere durch Kleben oder Verschweißen erzeugt.

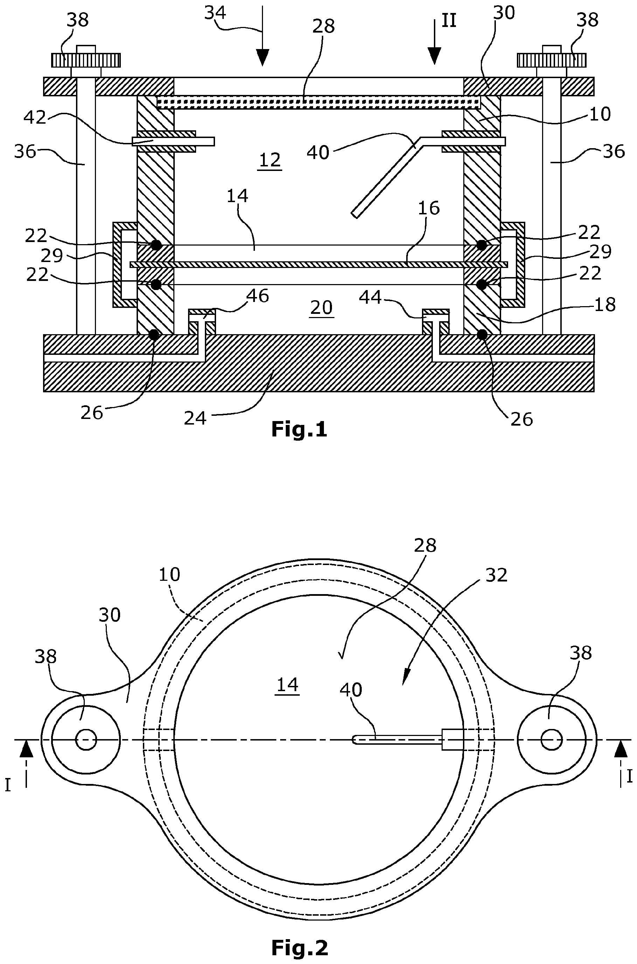

Weiter ist das erste Gehäuseteil 10 teilweise durch eine Abdeckung 30 überdeckt. Dabei hat die Abdeckung 30 eine Öffnung 32, wie in Fig. 2 zu sehen, so dass einfallendes Licht, dargestellt durch den Pfeil 34, durch die Abdeckung hindurchtreten kann, durch das Quarzglas 28 in den Hohlraum 12 gelangt und von dort den Prüfling 14 insbesondere vollflächig beleuchtet.

Mit der Abdeckung 30 sind zwei Befestigungselemente 36 verbunden, die in der dargestellten Ausführungsform als Schrauben ausgebildet sind. Die Befestigungselemente 36 sind ebenfalls mit dem Basiselement 24 verbunden. Durch zwei Rändelmuttern 38 werden das erste Gehäuseteil 10, das zweite Gehäuseteil 18 und dazwischen der Prüfling 14 zwischen der Abdeckung 30 und dem Basiselement 24 klemmend fixiert. Zum Ausbau des Prüflings 14 müssen lediglich die Rändelmuttern 38 gelöst werden, sodann die Abdeckung 30 sowie das erste Gehäuseteil 10 abgenommen werden.

Zur weiteren Abdichtung der Prüfungsapparatur ist eine Manschette 29 vorgesehen, die am ersten Gehäuseteil 10 und am zweiten Gehäuseteil 18 dicht anliegt und den Prüfling 14 dabei überdeckt.

Über eine Zuleitung 40 wird eine chemische Substanz oder ein Gemisch aus ausgewählten chemischen Substanzen in den Hohlraum 12 eingeleitet. Dabei ist die Zuleitung 40 in den Hohlraum 12 fortgesetzt und auf den Mittelpunkt des Prüflings 14 gerichtet. Weiter weist das erste Gehäuseteil 10 eine Ableitung 42 auf, über die die eingeleiteten chemischen Substanzen aus dem Hohlraum 10 abgeleitet werden können. Durch das Basiselement 24 wird eine Zuleitung 44 in den zweiten Hohlraum 20 geführt. Über eine Ableitung 46 werden Luftproben aus dem Hohlraum 20 abgeführt und zu einer nicht dargestellten Analyseeinrichtung transportiert.

Bei einer Überprüfung eines Schutzkleidungsmaterials 16 wird dieses in den Prüfling 14 eingespannt. Über die Zuleitung 40 werden chemische Substanzen in den Hohlraum 12 eingeleitet und über die Ableitung 42 wieder abgeleitet. Tritt eine chemische Substanz durch die zu prüfende Schutzkleidung 16 hindurch, so gelangt diese in den zweiten Hohlraum 20 und wird über die Ableitung 46 zu einer Analyseeinheit transportiert. Durch die Analyseeinheit wird festgestellt wann, wie viel und welche chemische Substanz das Schutzkleidungsmaterial durchdrungen hat. Weist das Schutzkleidungsmaterial 16 photokatalytische Substanzen auf, wird das Schutzkleidungsmaterial 16 während der Prüfung mit Licht einer bestimmten Wellenlänge beleuchtet.

## Patentansprüche

1. Prüfungsapparatur zur Prüfung der Rückhaltefähigkeit von Schutzkleidungsmaterialien (16), mit
einem einen ersten Hohlraum (12) ausbildenden ersten Gehäuseteil (10), einem einen zweiten Hohlraum (20) ausbildenden zweiten Gehäuseteil (18),
einem den ersten Hohlraum (12) des ersten Gehäuseteils (10) von dem zweiten Hohlraum (20) des zweiten Gehäuseteils (18) trennenden Prüfling (14) und
einer mindestens ein Befestigungselement (36) aufweisende Befestigungsvorrichtung zur Befestigung des Prüflings (14) zwischen dem ersten Gehäuseteil (10) und dem zweiten Gehäuseteil (18) wobei das erste Gehäuseteil (10) und/oder das zweite Gehäuseteil (18) mindestens zwei Anschlüsse als Zuleitung (40, 44) und Ableitung (42) aufweist,
**dadurch gekennzeichnet, dass**
die dem Prüfling gegenüberliegende Endfläche des ersten Gehäuseteils zumindest teilweise durch Quarzglas ausgebildet ist, um lichtdurchlässig und UV-durchlässig zu sein und
die Befestigungsvorrichtung eine das erste Gehäuseteil (10) zumindest teilweise überdeckende zumindest teilweise, der lichtdurchlässigen Endfläche des ersten Gehäuseteils entsprechende, lichtdurchlässige Abdeckung (30) aufweist.

2. Prüfungsapparatur nach Anspruch 1, mit ausschließlich dem ersten Gehäuseteil (10), dem zweiten Gehäuseteil (18), dem Prüfling (14), der Befestigungsvorrichtung und den mindestens zwei Anschlüssen.

3. Prüfungsapparatur nach Anspruch 1 oder 2, **dadurch gekennzeichnet, dass** das erste Gehäuseteil (10) und/oder das zweite Gehäuseteil (18) aus einem chemisch inerten Material, vorzugsweise Polytetrafluorethen hergestellt ist.

4. Prüfungsapparatur nach einem der Ansprüche 1 bis 3, **dadurch gekennzeichnet, dass** ein Anschluss des erste Gehäuseteils (10) auf die Mitte des Prüflings (14) gerichteten ist und insbesondere in den ersten Hohlraum (12) fortgesetzt ist.

5. Prüfungsapparatur nach einem der Ansprüche 1 bis 4, **dadurch gekennzeichnet, dass** das erste Gehäuseteil (10) oberhalb vom Prüfling (14) und dem zweiten Gehäuseteil (18) angeordnet ist und der Prüfling (14) oberhalb vom zweiten Gehäuseteil (18) angeordnet ist.

6. Prüfungsapparatur nach einem der Ansprüche 1 bis 5, **dadurch gekennzeichnet, dass** das mindestens eine Befestigungselement (36) flexibel ausgestaltet ist.

7. Prüfungsapparatur nach einem der Ansprüche 1 bis 6, **dadurch gekennzeichnet, dass** die Abdeckung (30) einteilig mit dem ersten Gehäuseteil (10) ausgebildet ist.

8. Prüfungsapparatur nach einem der Ansprüche 1 bis 7, **dadurch gekennzeichnet, dass** das mindestens eine Befestigungselement (36), mit der Abdeckung (30) verbunden ist.

9. Prüfungsapparatur nach einem der Ansprüche 1 bis 8, **dadurch gekennzeichnet, dass** das Befestigungselement (36) mit einem Basiselement (24) verbunden ist und insbesondere zwischen der Abdeckung (30) und dem Basiselement (24) der Prüfling (14) zwischen dem ersten Gehäuseteil (10) und dem zweiten Gehäuseteil (18) klemmend fixiert wird.

10. Prüfungsapparatur nach Anspruch 9, **dadurch gekennzeichnet, dass** der Abstand zwischen Abdeckung (30) und Basiselement (24) variabel ist zur klemmenden Fixierung eines Prüflings (14) unterschiedlicher Dicke zwischen dem ersten Gehäuseteil (10) und dem zweiten Gehäuseteil (18).

11. Prüfungsapparatur nach Anspruch 9 oder 10, **dadurch gekennzeichnet, dass** das zweite Gehäuseteil (18) einstückig mit dem Basiselement (24) ausgebildet ist.

12. Prüfungsapparatur nach einem der Ansprüche 9 bis 11, **dadurch gekennzeichnet, dass** die Anschlüsse des zweiten Gehäuseteils (18) durch das Basiselement (24) geführt werden.

13. Prüfungsapparatur nach einem der Ansprüche 9 bis 11, mit ausschließlich dem ersten Gehäuseteil (10), dem zweiten Gehäuseteil (18), dem Prüfling (14), dem Basiselement (24), der Befestigungsvorrichtung und den mindestens zwei Anschlüssen.

14. Prüfungsapparatur nach einem der Ansprüche 1 bis 13, **gekennzeichnet durch** eine das erste Gehäuseteil (10) mit dem zweiten Gehäuseteil (18) verbindende Dichtungsmanschette (22), die den Prüfling (14) vollständig überdeckt.

## Claims

1. A testing equipment for testing the sealing capacity of safety clothing materials (16), comprising
a first housing part (10) defining a first cavity (12),
a second housing part (18) defining a second cavity (20),
a test object (14) separating the first cavity (12) of the first housing part (10) from the second cavity (20) of the second housing part (18), and
a fastening device comprising at least one fastening element (36), for fastening the test object (14) between the first housing part (10) and the second housing part (18), the first housing part (10) and/or the second housing part (18) comprising at least two connecting ducts for use as feed (40, 44) and discharge passages (42),
**characterized in that**
the end face of the first housing part opposite the test object is at least partially made of quartz glass so as to be light-permeable and UV-permeable, and
the fastening device comprises a light-permeable cover (30) at least partially covering the first housing part (10) and at least partially corresponding to the light-permeable end face of the first housing part.

2. The testing equipment according to claim 1, comprising exclusively the first housing part (10), the second housing part (18), the test object (14), the fastening device and the at least two connecting ducts.

3. The testing equipment according to claim 1 or 2, **characterized in that** the first housing part (10) and/or the second housing part (18) are made of a chemically inert material, preferably polytetrafluoroethene.

4. The testing equipment according to any one of claims 1 to 3, **characterized in that** one connecting duct of the first housing part (10) is directed toward the center of the test object (14) and particularly is extended into the first cavity (12).

5. The testing equipment according to any one of claims 1 to 4, **characterized in that** the first housing part (10) is arranged above the test object (14) and the second housing part (18) and that the test object (14) is arranged above the second housing part (18).

6. The testing equipment according to any one of claims 1 to 5, **characterized in that** the at least one fastening element (36) is flexible.

7. The testing equipment according to any one of claims 1 to 6, **characterized in that** the cover (30) is formed integrally with the first housing part (10).

8. The testing equipment according to any one of claims 1 to 7, **characterized in that** the at least one fastening element (36) is connected to the cover (30).

9. The testing equipment according to any one of claims 1 to 8, **characterized in that** the fastening element (36) is connected to a base element (24) and that, particularly, between the cover (30) and the base element (24), the test object (14) is clampingly fixed between the first housing part (10) and the second housing part (18).

10. The testing equipment according to claim 9, **characterized in that** the distance between the cover (30) and the base element (24) is variable for clamping fixation of a test object (14) of varying thickness between the first housing part (10) and the second housing part (18).

11. The testing equipment according to claim 9 or 10, **characterized in that** the second housing part (18) is integrally formed with the base element (24).

12. The testing equipment according to any one of claims 9 to 11, **characterized in that** the connecting ducts of the second housing part (18) are guided through the base element (24).

13. The testing equipment according to any one of claims 9 to 11, comprising exclusively the first housing part (10), the second housing part (18), the test object (14), the base element (24), the fastening device and the at least two connecting ducts.

14. The testing equipment according to any one of claims 1 to 13, **characterized by** a sealing sleeve (22) connecting the first housing part (10) to the second housing part (18), said sealing sleeve fully covering the test object (14).

## Revendications

1. Appareil de test destiné à tester la capacité de retenue de matériau de revêtement et de protection (16), comprenant
une première partie de boîtier (10) formant une première cavité (12),
une seconde partie de boîtier (18) formant une seconde cavité (20),
un échantillon (14) séparant la première cavité (12) de la première partie de boîtier (10) de la seconde cavité (20) de la seconde partie de boîtier (18), et
un dispositif de fixation, comportant au moins un élément de fixation (36), qui est destiné à fixer l'échantillon (14) entre la première partie de boîtier (10) et la seconde partie de boîtier (18), la première partie de boîtier (10) et/ou la seconde partie de boîtier (18) comportant au moins deux raccords utilisée pour l'alimentation (40, 44) et la sortie (42),
**caractérisé en ce que** :
- la face d'extrémité, opposée à l'échantillon, de la première partie de boîtier est formée au moins partiellement par du verre de quartz pour être transparent à la lumière et perméable aux UV et
- le dispositif de fixation comporte un capot (30), transparent à la lumière, qui recouvre au moins partiellement la première partie de boîtier (10) et qui correspond au moins partiellement à la face d'extrémité, transparente à la lumière, de la première partie de boîtier.

2. Appareil de test selon la revendication 1, comprenant seulement la première partie de boîtier (10), la seconde partie de boîtier (18), l'échantillon (14), le dispositif de fixation et les au moins deux raccords.

3. Appareil de test selon la revendication 1 ou 2, **caractérisé en ce que** la première partie de boîtier (10) et/ou la seconde partie de boîtier (18) sont réalisées à partir d'un matériau chimiquement inerte, de préférence du polytétrafluoroéthylène.

4. Appareil de test selon l'une quelconque des revendications 1 à 3, **caractérisé en ce qu'**un raccord de la première partie de boîtier (10) est dirigé vers le milieu de l'échantillon (14) et se poursuit en particulier dans la première cavité (12).

5. Appareil de test selon l'une des revendications 1 à 4, **caractérisé en ce que** la première partie de boîtier (10) est disposée au-dessus de l'échantillon (14) et de la seconde partie de boîtier (18) et l'échantillon (14) est disposé au-dessus de la seconde partie de boîtier (18).

6. Appareil de test selon l'une des revendications 1 à 5, **caractérisé en ce que** l'élément de fixation (36) est formé de manière flexible.

7. Appareil de test selon l'une des revendications 1 à 6, **caractérisé en ce que** le couvercle (30) est formé d'une seule pièce de la première partie de boîtier (10).

8. Dispositif de test selon l'une des revendications 1 à 7, **caractérisé en ce que** l'au moins élément de fixation (36) est relié au couvercle (30).

9. Appareil de test selon l'une des revendications 1 à 8, **caractérisé en ce que** l'élément de fixation (36) est relié à un élément de base (24) et est fixé par serrage entre la première partie de boîtier (10) et la seconde partie de boîtier (18) en particulier entre le couvercle (30) et l'élément de base (24) de l'échantillon (14).

10. Appareil de test selon la revendication 9, **caractérisé en ce que** la distance entre le couvercle (30) et l'élément de base (24) est variable pour fixer par serrage un échantillon (14) d'épaisseurs différentes entre la première partie de boîtier (10) et la seconde partie de boîtier (18).

11. Appareil de test selon la revendication 9 ou 10, **caractérisé en ce que** la seconde partie de boîtier (18) est formée d'une seule pièce avec l'élément de base (24).

12. Appareil de test selon l'une des revendications 9 à 11, **caractérisé en ce que** les raccords de la seconde partie de boîtier (18) sont guidés à travers l'élément de base (24).

13. Appareil de test selon l'une des revendications 9 à 11, comportant seulement la première partie de boîtier (10), la seconde partie de boîtier (18), l'échantillon (14), l'élément de base (24), le dispositif de fixation et les au moins deux raccords.

14. Appareil de test selon l'une des revendications 1 à 13, **caractérisé par** un manchon d'étanchéité (22) qui relie la première partie de boîtier (10) à la seconde partie de boîtier (18) et qui recouvre complètement l'échantillon (14).
